# EUROPÄISCHE PATENTSCHRIFT

(11) **EP 3 614 141 B1**
(45) Veröffentlichungstag und Bekanntmachung des Hinweises auf die Patenterteilung: **05.10.2022**
(21) Anmeldenummer: 19193034.6
(22) Anmeldetag: 22.08.2019
(51) Int. Cl.: G01N 31/22, A61L 15/56, G01N 21/78

(54) **STABILER URIN-INDIKATOR MIT LANGZEITDETEKTION UND VERFAHREN ZUR HERSTELLUNG**
STABLE URINE INDICATOR WITH LONG-TERM DETECTION AND METHOD FOR ITS PREPARATION
INDICATEUR URINAIRE STABLE AVEC DÉTECTION À LONG TERME ET PROCÉDÉ DE FABRICATION

(30) Priorität: 23.08.2018 DE 102018214263
(43) Veröffentlichungstag der Anmeldung: 26.02.2020
(73) Patentinhaber: AXAGARIUS GmbH & Co. KG, 52355 Düren (DE)
(72) Erfinder: Husmann, Ralph, 52355 Düren (DE); Hoffmann, Jürgen, 52355 Düren (DE)
(74) Vertreter: dompatent von Kreisler Selting Werner - Partnerschaft von Patent- und Rechtsanwälten mbB

(56) Entgegenhaltungen:
- WO-A2-2009/133480
- WO-A2-2011/007290
- DE-A1- 1 698 247
- US-A1- 2013 066 289
- US-A1- 2014 087 181

## Beschreibung

Die vorliegende Erfindung betrifft eine Testvorrichtung zum pH-abhängigen Nachweis von Urin. Die Erfindung betrifft zudem einen Hygieneartikel umfassend die erfindungsgemäße Testvorrichtung und ein Verfahren zur Herstellung der erfindungsgemäßen Testvorrichtung.

Urin ist ein flüssiges Ausscheidungsprodukt des Menschen, für das gerade im Hygienebereich (Windeln, Inkontinenzartikel) ein einfacher und schneller Nachweis von Vorteil ist.

Die US 4,022,211 schlägt für Windeln einen Urin-Nachweis über einen Nässeindikator vor. Dieser Nässeindikator weist einen wasserlöslichen Farbstoff auf, der durch den Urin gelöst wird und mit diesem in die absorbierende Lage der Windel wandert. Die sich dadurch anfärbende absorbierende Lage dient als Urin-Nachweis. Dieser Nachweis hat den Nachteil, dass er wenig sensitiv ist, denn es muss ausreichend Farbstoff gelöst und weiter transportiert werden, um eine klare Windelanfärbung zu erzielen. Weiterhin ist die Testvorrichtung nicht stabil, ein transientes Feuchtwerden während Herstellung, Lagerung oder Transport "aktiviert" bereits den Nässeindikator. Beachtlicherweise ist dieser Nachweis auch nicht selektiv, insofern auch einfaches Wasser als Urin detektiert werden würde.

Die DE 1698247 offenbart die Herstellung einer cellulosehaltigen Trägermatrix mit einem durch kovalente chemische Bindung an die Cellulose immobilisierten pH-Indikatorfarbstoff und die Verwendung derselben zur Bestimmung von pH-Werten, z.B. von wässrigen Lösungen, Abwässern, Körperflüssigkeiten, etc. Die kovalente Bindung des pH-Indikatorfarbstoffs an die cellulosehaltige Trägermatrix erfolgt durch Einführung einer reaktiven Gruppe in das pH-Indikatorfarbstoffmolekül (z.B. einer 2-(Hydroxysulfonyloxy)-ethylsulfonyl-Gruppe) und dessen Reaktion mit der cellulosehaltigen Trägermatrix unter Einwirkung einer Base (z.B. Natriumcarbonat und Natronlauge) mit anschließendem Neutralwaschen mit Wasser gefolgt von einem Trocknungsschritt.

Ein selektiver Urin-Nachweis ist auch deshalb schwer zu führen, weil Urin in seiner Zusammensetzung eine hohe Variabilität aufweist:
- Der pH-Wert kann zwischen 4,6 und 7,4 betragen.
- Die Osmolarität kann zwischen 50 und 1200 mosmol/L variieren.
- Die Farbe kann zwischen Farblos und Orangerot variieren.
- Der Urin kann weitere Bestandteile wie Proteine, Aminosäuren, Nitrit, Leukozyten, Ascorbinsäure, Ketonkörper, Urobilinogen, Bilirubin, Hämoglobin oder Glucose enthalten.
- Der Urin kann in seiner Dichte variieren.

Es besteht daher ein Bedarf an verbesserten Verfahren und Vorrichtungen zum Nachweis von Urin.

Aufgabe der Erfindung ist es daher, eine Testvorrichtung für den Nachweis von Urin dahingehend zu verbessern, dass sie bezüglich mindestens einer der oben genannten Nachteile verbessert wird.

Diese Aufgabe wird gemäß der Erfindung durch den Gegenstand der unabhängigen Ansprüche 1 bis 3 gelöst. Spezifische Ausführungsformen der Erfindung sind Gegenstand der zusätzlichen abhängigen oder unabhängigen Ansprüche.

### Zusammenfassung der Erfindung

In einem ersten Aspekt stellt die Erfindung eine Testvorrichtung zum pH-abhängigen Nachweis von Urin bereit, wobei die Testvorrichtung eine cellulosehaltige Trägermatrix enthält, die Folgendes aufweist:
a) einen pH-Indikatorfarbstoff mit einem Umschlagspunkt zwischen pH=0 und pH=4,5, der mittels einer chemischen Bindung an die Cellulose immobilisiert ist und mit einer Menge einer feststofflichen Säure so vorbehandelt ist, dass der pH-Indikatorfarbstoff im trockenen Zustand die Farbe des sauren pH-Bereiches anzeigt;
   oder
b) einen pH-Indikatorfarbstoff mit einem Umschlagspunkt zwischen pH=7,5 und pH=14, der mittels einer chemischen Bindung an die Cellulose immobilisiert ist und mit einer Menge einer feststofflichen Base so vorbehandelt ist, dass der pH-Indikatorfarbstoff im trockenen Zustand die Farbe des basischen pH-Bereiches anzeigt, wobei der pH-Indikatorfarbstoff ein Reaktivfarbstoff ist mit mindestens einer reaktiven Gruppe zur kovalenten Anbindung an die Cellulose und zusätzlich zu der mindestens einen reaktiven Gruppe mindestens eine Sulfonsäure- und/oder Carbonsäuregruppe aufweist.

Die Testvorrichtung der Erfindung ist in Anspruch 1 definiert.

Unter der Vorbehandlung des mittels einer chemischen Bindung an die Cellulose immobilisierten pH-Indikatorfarbstoffs versteht man die Behandlung (d.h. die Umsetzung) des auf der Cellulose immobilisierten pH-Indikatorfarbstoffs mit einer feststofflichen Säure bzw. feststofflichen Base in einer solchen Menge, dass der pH-Indikatorfarbstoff im trockenen Zustand die Farbe des sauren bzw. basischen pH-Bereiches anzeigt. Durch den Kontakt mit Urin wird der Farbwechsel rückgängig gemacht, ein Farbwechsel bei Kontakt mit Wasser alleine findet nicht statt.

Die erfindungsgemäße Testvorrichtung vereinigt mehrere entscheidende Vorteile gegenüber den aus dem Stand der Technik bekannten Testvorrichtungen.

Ein wichtiger Vorteil besteht in der Selektivität der Nachweisreaktion: Wie oben bereits ausgeführt, kann nur der Urin als gepufferte Flüssigkeit mit einem pH zwischen 4,5 und 7,5 den pH-induzierten Farbwechsel bewirken. Durch Benetzen der Testmatrix mit Wasser wird lediglich die immobilisierte Säure bzw. Base partiell oder vollständig gelöst und der vorab durch diese Substanzen eingestellte pH-Wert ändert sich nicht. Dadurch bleibt auch die initiale Farbe der Testmatrix erhalten.

Durch diese Selektivität weist die Testvorrichtung auch eine hohe Stabilität hinsichtlich Produktion und Lagerung auf. Sollte es im Herstellungsverfahren oder während der Lagerung zu einer erhöhten Feuchtigkeitsaufnahme der Testvorrichtung kommen, so kommt es zu keinem Farbwechsel und die Testvorrichtung bleibt in ihrer aktivierten Form und kann nach Trocknen oder sogar in feuchtem Zustand noch verwendet werden.

Da die Testvorrichtung auf einer schnellen Säure-Base-Reaktion mit direktem Farbnachweis beruht, stellt sie ein direktes und sehr schnelles Nachweisverfahren dar.

Zudem ist die erfindungsgemäße Testvorrichtung einfach zu gebrauchen und zu interpretieren und bedarf keiner zusätzlichen Messgeräte. Gerade für die Verwendung bei Windeln oder Inkontinenzartikeln erhöht dies die Compliance, da der Farbwechsel schnell und unkompliziert zu detektieren ist.

Wie die Erfinder festgestellt haben, führt die Testvorrichtung bei dem Urin-Nachweis auch zu einem stabilen Testergebnis, das nach dem initialen Urin-induzierten Farbwechsel lange anhält. Diese Stabilität beruht auf dem immobilisierten pH-Indikatorfarbstoff, der seine kräftige Farbe behält, also nicht verblasst und auch kein "Ausbluten" zeigt.

Von besonderer Wichtigkeit ist hier, dass das Testergebnis auch bei einer transienten Urin-Anwesenheit ein stabiles Testergebnis zeigt. Sollte also die Testmatrix nur kurzzeitig mit Urin in Kontakt kommen und dadurch einen Farbwechsel erzeugen, so bleibt dieser Farbwechsel auch nach anschließendem Trocknen der Testmatrix erhalten. Durch den Trocknungsprozess wird der Urin als Reaktionskomponente in der Testmatrix soweit "immobilisiert", dass die Testmatrix weiterhin in den Urin-induzierten pH-Wechsel anzeigt.

Die Testvorrichtung ist im Messverhalten sehr robust: Andere Urinbestandteile, wie beispielsweise Proteine, Ketonkörper oder Glucose stören die Messung nicht.

Die Testvorrichtung weist auch eine ausreichende Stabilität auf und bedarf bei Verwendung der herkömmlichen Reagenzien auch keiner Kühlung.

Die Testvorrichtung kann mit handelsüblichen Substanzen auf einfache Weise und zudem kostengünstig hergestellt werden.

Die Testvorrichtung kann ohne großen Mehraufwand in bereits etablierte Hygieneartikel, wie Windeln oder Inkontinenzartikel integriert werden.

Der Fachmann kann hinsichtlich der einzelnen Komponenten auf eine Vielzahl an chemischen Substanzen zurückgreifen und die Vorrichtung so gezielt an die jeweilige Anwendung anpassen. Insbesondere die Auswahl der feststofflichen Säure/Base und des pH-Indikators erlauben eine Anwendung für verschiedene Nachweisstrategien.

### Ausführliche Beschreibung der Erfindung

Die Testvorrichtung basiert in der ersten "aziden" Ausführungsform auf einer Hochtitration des pH-Indikators, der durch vorab co-immobilisierte azide wirkenden Gruppen (seien sie extern als Feststoff hinzugegeben, von dem pH-indikator bereitgestellt oder in der Matrix enthalten) den sauren pH-Bereich anzeigt und durch die Urin-induzierte pH-Werterhöhung über den Umschlagsbereich gelangt, und so die dem neutralen/basischen Milieu entsprechende Farbe anzeigt.

Die Testvorrichtung basiert in der zweiten "basischen" Ausführungsform auf einer Heruntertitration des pH-Indikators, der durch vorab co-immobilisierte basisch wirkende Gruppen (seien sie extern als Feststoff hinzugegeben, von dem pH-indikator bereitgestellt oder in der Matrix enthalten) den basischen pH-Bereich anzeigt und durch die Urin-induzierte pH-Werterniedrigung über den Umschlagsbereich gelangt, und so die dem aziden/neutralen Milieu entsprechende Farbe anzeigt.

Erfindungsgemäß wird bei der Testvorrichtung eine cellulosehaltige Trägermatrix verwendet, die als Träger für den immobilisierten pH-Indikatorfarbstoff dient. In einer Ausführungsform umfasst diese Trägermatrix die Cellulose, sie kann aber neben der Cellulose auch andere Materialien enthalten. Bevorzugt besteht die cellulosehaltige Trägermatrix im Wesentlichen aus Cellulose oder gänzlich aus Cellulose. Die Cellulose stellt einen essentiellen Bestandteil der Trägermatrix dar, insofern sie als Bindungspartner für den pH-Indikatorfarbstoff dient.

Die erfindungsgemäße Trägermatrix ist zweckmäßigerweise aus einem cellulosehaltigen Trägermaterial, das den Durchtritt von Flüssigkeiten erlaubt. Erfindungsgemäß handelt es sich hierbei insbesondere um poröse Materialien, die bevorzugterweise die Flüssigkeit aufsaugen und so eine definierte Menge an Flüssigkeit in Reaktion mit den Nachweisreagenzien bringen.

Dem Fachmann sind aus dem Stand der Technik zahlreiche Materialien und Strukturen bekannt die als Trägermatrix geeignet sind und er kann in Abhängigkeit von der konkreten Verwendung gezielt auswählen.

In einer bevorzugten Ausführungsform ist die cellulosehaltige Trägermatrix ausgewählt aus der Gruppe bestehend aus Papier, bevorzugt Filterpapier; Karton; textiles Gewebe; textiles Vlies; und textiles Gewirke.

In einer besonders bevorzugten Ausführungsform handelt es sich bei der cellulosehaltigen Trägermatrix um Filterpapier. Filterpapiere sind kostengünstig und besitzen eine gute Saugfähigkeit und eine hohe Aufnahmekapazität für Flüssigkeiten. Sie können daher in einfacher Weise, beispielsweise durch Tränken und anschließendes Trocknen mit der Säure oder Base imprägniert werden und auch im eigentlichen Test schnell und effektiv von dem Urin durchtränkt werden. Zudem können Sie in einfacher Weise auf die gewünschte Form geschnitten werden und mit anderen Bestandteilen eines Hygieneartikels verbunden werden (z.B. durch eine Klebeverbindung).

In einer Ausführungsform ist die Trägermatrix einschichtig aufgebaut, so dass alle Nachweisreagenzien in dieser Schicht enthalten sind. In einer alternativen Ausführungsform kann die Trägermatrix aus zwei oder mehr Schichten aufgebaut sein. Die einzelnen Schichten können so beispielsweise eine unterschiedliche Saugfähigkeit bzw. Aufnahmekapazität für Flüssigkeiten aufweisen, so dass die flüssige Probe gezielter aufgenommen werden kann und auch ein Ausbluten der Trägermatrix verhindert werden kann. Zudem erlaubt dies eine räumliche Trennung der unterschiedlichen Nachweisreagenzien, so dass chemisch/physikalisch nicht-kompatible Nachweisreagenzien verwendet werden können, oder die von außen eindringende flüssige Probe beim Durchdringen der einzelnen Schichten sequentiell mit den Nachweisreagenzien reagieren kann.

Weiterhin kann die Trägermatrix auch einen als "Waste-Pad" ("Abfall-Pad") bezeichneten Bereich aufweisen, der die durch die Trägermatrix durchgelaufene Flüssigkeit aufnimmt. In diesem Bereich kann z.B. eine saugfähige Matte, bzw. ein Vlies, ein Lösch- oder Filterpapier oder dergleichen vorgesehen sein.

Die Ausgestaltung der Trägermatrix kann in Form und Tiefe derart erfolgen, dass eine kleine Chromatographiesäule entsteht, bei der eventuell störende Probenbestandteile abgetrennt werden können.

Erfindungsgemäß wird der pH-Indikatorfarbstoff durch eine mit der Cellulose eingegangene chemische Bindung an diese Cellulose gebunden und somit an die Trägermatrix immobilisiert. Die Immobilisierung und die ihr zugrundeliegende chemische Bindung ist dadurch definiert, dass es bei Einwirken wässriger Flüssigkeiten zu keiner wesentlichen Auflösung der Bindung und damit zu keiner Beeinträchtigung der Immobilisierung kommt. Der Fachmann spricht hier von farbfixierten Trägern (z.B. also von farbfixierten pH-Indikatorpapieren), die dementsprechend als "nichtblutende" Testvorrichtungen bezeichnet werden.

Erfindungsgemäß handelt es sich bei der chemischen Bindung zur Immobilisierung des pH-Indikatorfarbstoffs an die Cellulose um eine ionische Bindung, eine kovalente Bindung oder eine van-der-Waals-Bindung.

Hierbei ist eine kovalente Bindung, wie sie beispielsweise von Reaktivfarbstoffen eingegangen wird, bevorzugt, da sie mit einer sicheren und dauerhaften Immobilisierung an der Trägermatrix einhergeht.

In einer bevorzugten Ausführungsform ist der an der Trägermatrix immobilisierte pH-Indikator ein Reaktivfarbstoff.

Dem Fachmann sind zahlreiche, als pH-Indikatoren wirkende Reaktivfarbstoffe bekannt. Diese können ausgewählt sein aus der Gruppe aufweisend Azofarbstoff, Anthrachinon-Farbstoff, Triphenylmethan-Farbstoff und Anthocyan-Farbstoff. Bevorzugt ist hierbei die Verwendung von Azofarbstoffen.

Für die Bestimmung des Urins ausgehend vom Säure-behandelten pH-Indikatorfarbstoff ist es erfindungsgemäß vorgesehen, dass der pH-Indikatorfarbstoff einen Umschlagspunkt aufweist, der zwischen pH = 0 und pH = 4,5 liegt. Bevorzugt ist hier einen Umschlagspunkt zwischen pH = 0,5 und pH = 4,0 und besonders bevorzugt zwischen pH = 1,0 und pH = 3,5.

Für die Bestimmung des Urins ausgehend vom alkalisch-behandelten pH-Indikatorfarbstoff ist es erfindungsgemäß vorgesehen, dass der pH-Indikatorfarbstoff einen Umschlagspunkt aufweist, der zwischen pH = 7,5 und pH = 14 liegt. Bevorzugt ist hier ein Umschlagspunkt zwischen pH = 8,0 und pH = 13,5 und besonders bevorzugt zwischen pH = 8,5 und pH = 13,0.

Zur kovalenten Anbindung an die Cellulose weist der Reaktivfarbstoff mindestens eine reaktive Gruppe auf. Diese mindestens eine reaktive Gruppe ist bevorzugt ausgewählt aus der Gruppe bestehend aus 2-(Hydroxysulfonyloxy)-ethylsulfonyl, *N*-Methyl-*N*-[2-(hydroxysulfonyloxy)-ethyl]sulfonamido, Monochlortriazinyl, Dichlortriazinyl, Monochlorpyrimidyl, Dichlorpyrimidyl, Trichlorpyrimidyl, Tetrachlorpyrimidyl, Dichlorpyridazinyl, Trichlorpyridazinyl, Tetrachlorpyridazinyl, Dichlorchinoxalinyl, Dichlorphthalazinyl, 2-(Hydroxysulfonyloxy)ethylaminosulfonyl, 2,3-Dichlorchinoxalin-6-carbonsäure, 4-Chlorbenzolsulfonsäure [2-methoxyethylamid], 2-Chlorchinoxalin-6-carbonsäure, 3-Chlorchinoxalin-6-carbonsäure, 2,3-Dichlorchinoxalin-6-sulfonsäure, 2,3-Dichlorphthalazin-6-carbonsäure, 2,4-Dichlor-1,3,5-triazinyl, 2,4,6-Trichlor-1,3,5-triazinyl, 2,4-Dichlor-6-benzo-1,3,5-triazinyl, 2,4-Dichlor-6-amino-1,3,5-triazinyl und 3,5,6-Trichlor-1,2,4-triazinyl.

Erfindungsgemäß weist der Reaktivfarbstoff zusätzlich zu der mindestens einen reaktiven Gruppe mindestens eine Sulfonsäure- und/oder Carbonsäuregruppe auf. Diese Gruppen erhöhen die Löslichkeit des Reaktivfarbstoffs und erleichtern bei dem Verfahren zur Anbindung des Reaktivfarbstoffs an die Cellulose das Auswaschen überschüssiger Farbstoffmoleküle oder Reaktionsnebenprodukte.

Die feststoffliche Säure der Testvorrichtung dient dazu, bei dem pH-Indikatorfarbstoff einen Farbumschlag in den aziden Bereich zu induzieren und diese, den aziden Bereich anzeigende Farbe bis zur Reaktion mit dem Urin aufrechtzuerhalten. Insofern die feststoffliche Säure als Feststoff vorliegt, verflüchtigt sie sich nicht, sondern verbleibt als Feststoff in der Trägermatrix und erlaubt eine langzeitstabile Formulierung.

In einer bevorzugten Ausführungsform ist die feststoffliche Säure ausgewählt aus der Gruppe bestehend aus Ascorbinsäure, Adipinsäure, Äpfelsäure, Agaricinsäure, Amidoschwefelsäure, 4-Aminosalicylsäure, Asparaginsäure, Bernsteinsäure, Benzolsulfonsäure, Benzoesäure, Borsäure, Caprinsäure, Cyclaminsäure, 2,2-Dichloressigsäure, Embonsäure, Ethansulfonsäure, Ethandisulfonsäure, Fumarsäure, Gentisinsäure, Gluconsäure, Glucuronsäure, Glutaminsäure, Glutarsäure, Glycerinsäure, Glycolsäure, Hippursäure, 2-Hydroxyethansulfonsäure, α-Ketoglutarsäure, Lactobionsäure, Laurinsäure, Maleinsäure, Malonsäure, Mandelsäure, Milchsäure, Mucinsäure, Natriumhydrogensulfat, Oxalessigsäure, Oxalsäure, Phthalsäure, 2-Phosphogylcerinsäure, 3-Phosphogylcerinsäure, Propionsäure, Polystyrolsulfonsäure, Pyroglutaminsäure, Pyrrolidin-2-carbonsäure, Salicylsäure, Sebacinsäure, Sorbinsäure, Sulfamsäure, *p*-Toluolsulfonsäure, Weinsäure, Zimtsäure und Zitronensäure.

In einer bevorzugten Ausführungsform handelt es sich bei der Säure um eine physiologisch verträgliche und damit pharmazeutisch akzeptable Substanz, wie Zitronensäure, Ascorbinsäure oder Benzoesäure.

In besonders bevorzugter Weise handelt es sich bei der feststofflichen Säure um Zitronensäure.

Für die Bestimmung des Urins mit einem pH-Wert von 4,5 bis 7,5 ist es bevorzugt, dass die feststoffliche Säure einen pKs-Wert zwischen 1,0 und 5,0 aufweist.

Die feststoffliche Base der Testvorrichtung dient dazu, bei dem pH-Indikatorfarbstoff einen Farbumschlag in den basischen Bereich zu induzieren und diese, den basischen Bereich anzeigende Farbe bis zur Reaktion mit dem Urin aufrechtzuerhalten. Insofern die feststoffliche Base als Feststoff vorliegt, verflüchtigt sie sich nicht, sondern verbleibt als Feststoff in der Trägermatrix und erlaubt eine langzeitstabile Formulierung.

In einer bevorzugten Ausführungsform ist die feststoffliche Base ausgewählt aus der Gruppe bestehend aus Natriumhydroxid, Kaliumhydroxid, Lithiumhydroxid, Magnesiumhydroxid, Calciumhydroxid, Natriumphosphat, Natriumcarbonat und Natriumsulfid, wobei Natriumhydroxid bevorzugt ist.

In einer bevorzugten Ausführungsform handelt es sich bei der Base um eine physiologisch verträgliche und damit pharmazeutisch akzeptable Substanz, wie Natriumhydroxid oder Kaliumhydroxid.

In besonders bevorzugter Weise handelt es sich bei der feststofflichen Base um Natriumhydroxid.

Für die Bestimmung des Urins mit einem pH-Wert von 4,5 bis 7,5 ist es bevorzugt, dass die feststoffliche Base einen pKb-Wert zwischen -2,0 und 4,0 aufweist.

In einer Ausführungsform der Erfindung ist die Testvorrichtung als Teststreifen, rechteckiges Testfeld oder Testband ausgebildet oder so ausgestaltet, dass sie in ein integriertes Testsystem aufnehmbar ist.

Der Teststreifen kann aus einer Vielzahl von Materialien hergestellt werden. Bevorzugt sind hierbei wasserfeste Materialien wie Kunststoffe. Der Teststreifen besteht hierbei bevorzugt aus Polyvinylchlorid oder Polyethylen.

In einer weiteren Ausführungsform kann die Testvorrichtung weitere Trägermatrizes aufweisen.

Durch eine oder mehrere zusätzliche Trägermatrizes können weitere Urin-Parameter quantitativ oder qualitativ bestimmt werden, wie pH-Wert, Dichte, Protein, Glucose, Leukocyten, Nitrit, Hämoglobin, Urobilinogen, Bilirubin oder Ketonkörper.

Zweckmäßigerweise kann die Trägermatrix auch zum qualitativen oder quantitativen Nachweis anderer Urinbestandteile ausgestaltet sein.

In einer Ausführungsform der Erfindung ist die vorgenannte Trägermatrix auf einem Teststreifen aufgebracht.

In einem zweiten Aspekt stellt die Erfindung ein Testverfahren zum Urin-Nachweis bereit, dass die folgenden Schritte umfasst:
a) Tränken der Trägermatrix mit der flüssigen Probe, bevorzugt durch Eintauchen in die Probe;
b) Entfernen überschüssigen Probenmaterials von der Trägermatrix, bevorzugt durch Herausziehen des Teststreifens aus der Probe;
c) Optional eine Inkubation des Teststreifens für mindestens 5 Sekunden, bevorzugt bei Raumtemperatur (21 °C);
d) Visuelles Erfassen des Farbwertes der Trägermatrix.

In einem zweiten Aspekt betrifft die Erfindung einen Hygieneartikel, der eine oder mehrere der erfindungsgemäßen Testvorrichtungen umfasst.

Zweckmäßigerweise wird die Testvorrichtung in Hygieneartikeln eingesetzt, die gemäß ihrer jeweiligen Anwendung mit Urin in Kontakt kommen können. In bevorzugter Weise ist dieser Hygieneartikel ausgewählt aus der Gruppe bestehend aus Windel, Windeleinlage, Inkontinenzartikel wie Inkontinenzeinlage, Inkontinenzauflage oder Inkontinenzhose und Bettauflage.

In besonders bevorzugter Weise ist der Hygieneartikel eine Windel und speziell eine Einwegwindel.

In einem dritten Aspekt betrifft die Erfindung ein Verfahren zur Herstellung der erfindungsgemäßen Testvorrichtung, wobei das Verfahren die folgenden Schritte umfasst:
a) Bereitstellen einer cellulosehaltigen Trägermatrix mit einem durch chemische Bindung an die Cellulose immobilisierten pH-Indikatorfarbstoff,
b) Imprägnieren der farbstofftragenden Cellulose-Trägermatrix aus Schritt a) durch Durchtränken mit einer Imprägnierlösung, die Wasser, Alkohol und eine feststoffliche Base oder eine feststoffliche Säure enthält,
c) Trocknen der farbstofftragenden Cellulose-Trägermatrix aus Schritt b),
d) Optionales Zurechtschneiden der getrockneten Trägermatrix aus Schritt c) auf die gewünschte Größe.

Hierbei ist es bevorzugt, dass die im Schritt (b) eingesetzte Imprägnierlösung die Säure oder Base in einer Konzentration zwischen 2 mM und 75 mM, bevorzugt zwischen 5 und 60 mM und besonders bevorzugt zwischen 10 und 30 mM enthält.

Das Trocknen im Schritt c) des Herstellungsverfahrens erfolgt bevorzugt bei einer Temperatur von mehr als 25 °C und besonders bevorzugt bei einer Temperatur zwischen 250 und 350 °C.

Durch die Schritte b) und c) erfolgt die (Vor-) Behandlung des an die Cellulose mittels einer chemischen Bindung immobilisierten pH-Indikatorfarbstoffs mit der feststofflichen Base oder der feststofflichen Säure so, dass der pH-Indikatorfarbstoff im trockenen Zustand die Farbe des basischen oder sauren pH-Bereichs anzeigt.

Die cellulosehaltige Trägermatrix mit einem durch chemische Bindung an die Cellulose immobilisierten pH-Indikatorfarbstoff gemäß Schritt a) kann beispielsweise wie in der DE 1698247 beschrieben, hergestellt werden.

### Definitionen

Unter einer "Testvorrichtung" werden im Rahmen der vorliegenden Erfindung alle trägergebundenen Tests für medizinische und nichtmedizinische Zwecke verstanden. Bei diesen trägergebundenen Tests sind Nachweisreagenzien in der Trägermatrix eines Trägers eingebettet, der mit der flüssigen Probe in Kontakt gebracht wird. Die Reaktion von flüssiger Probe und Reagenzien führt bei Anwesenheit des Zielanalyten, also hier des Urins, zu einem nachweisbaren Signal, nämlich einem Farbumschlag, welcher visuell oder mit Hilfe eines Geräts beispielsweise transmissionsphotometrisch, reflexionsphotometrisch oder fluoreszenzphotometrisch ausgewertet werden kann.

Gemäß der Erfindung ist unter einer "feststofflichen Säure" eine Säure zu verstehen, die bei Raumtemperatur (21 °C) in einem festen Aggregatzustand vorliegt. Dies kann sowohl eine organische Säure als auch eine anorganische Säure sein.

Gemäß der Erfindung ist unter einer "feststofflichen Base" eine Base zu verstehen, die bei Raumtemperatur (21 °C) in einem festen Aggregatzustand vorliegt. Dies kann sowohl eine organische Base als auch eine anorganische Base sein.

Im Rahmen der Erfindung ist unter einem "pH-Indikator" eine Substanz zu verstehen, die in Abhängigkeit von dem pH-Wert ihre Farbe ändert.

Unter einem Reaktivfarbstoff soll im Sinne der Anmeldung ein Farbstoff zum Färben von Cellulose verstanden werden. Beim Färbeprozess bildet sich eine kovalente chemische Bindung zwischen dem Farbstoff und den funktionellen Gruppen der Cellulose aus.

Unter "Selektivität" versteht man die Fähigkeit bestimmter Substanzen, aus einer Anzahl gebotener Möglichkeiten zur Reaktion eine bevorzugt auszusuchen. Die ausschließliche Auswahl bezeichnet man als Spezifität.

Die "Stabilität" der Testvorrichtung beinhaltet Lagerstabilität, Stabilität unter physikalischen Einflüssen wie z.B. Wärme, Licht, mechanische Beanspruchung.

### Ausführungsbeispiele

### 1. Herstellung einer Urin-Testvorrichtung

Es wird eine Imprägnierlösung gemäß der folgenden Rezeptur hergestellt:

| Inhaltsstoffe | Menge |
|---|---|
| Zitronensäure | 5,0 g |
| Ethanol | 500 mL |
| VE-Wasser | 500 mL |

Als Trägermatrix wird ein Filterpapier mit einem immobilisierten pH-Indikatorfarbstoff verwendet (sog. nichtblutendes oder farbfixiertes pH-Indikatorpapier).

Zur Imprägnierung wird das farbfixierte pH-Indikatorpapier in die Imprägnierlösung eingetaucht und anschließend bei 325 °C für 60 Sekunden getrocknet. Das Papier kann anschließend im gewünschten Format zurechtgeschnitten werden.

### 2. Urin-Nachweisreaktion mit verschiedenen Indikatorfarbstoffen

Um pH-Indikatorfarbstoffe zu identifizieren, die einen besonders klaren Farbumschlag zeigen, wurden vier gemäß Beispiel 1 hergestellte Testpapiere basierend auf vier unterschiedlichen Indikatorfarbstoffen mit Urin getränkt und der Farbwechsel visuell erfasst. Das Ergebnis ist in der folgenden Tabelle 1 dargestellt.

**Tab. 1. Übersicht von farbfixierten pH-Indikatorpapier mit einem Farbumschlag im sauren Bereich**

| **Eintrag** | **pH Umschlagsbereich** | **Farbänderung** |
|---|---|---|
| **1** | 0,0 - 3,5 | Magenta - Gelb |
| **2** | 0,0 - 4,0 | Violett - Orange |
| **3** | 1,0 - 5,0 | Rot - Gelb |
| **4** | 1,5 - 4,5 | Blau - Magenta |

Die Papiere zeigen im sauren pH-Bereich Farbumschläge von Magenta nach Gelb (Eintrag 1), Violett nach Orange (Eintrag 2), Rot nach Gelb (Eintrag 3) oder Blau nach Magenta (Eintrag 4). Der Farbumschlag von Blau nach Magenta (Eintrag 4) ist besonders gut zu unterscheiden und weitere Versuche wurden mit diesem Papier durchgeführt.

### 3. Abhängigkeit der Nachweisreaktion von der Pufferstärke der Testlösung

Für den Urin-Nachweis ist es notwendig, dass die Testvorrichtungen auch bei einer Urinprobe mit sehr geringer Osmolarität einen eindeutigen Urin-Nachweis erlaubt. Die Osmolarität von Urin liegt typischerweise zwischen 600 und 900 mosmol/L. Die Osmolarität kann aber in Abhängigkeit vor allem von Flüssigkeitszufuhr und Flüssigkeitsverlusten zwischen 50 und 1200 mosmol/L variieren. Entsprechend ist ein Nachweis einer 25 mM Pufferlösung notwendig, um auch stark hypoosmolaren Urin mit 50 mosmol/L eindeutig detektieren zu können.

Um die Mindestpuffermenge zu bestimmen, bei der das sauer eingestellte Papier seine Farbe ändert, wird das Papier gemäß Eintrag 4 (Farbwechsel von Blau nach Magenta) zuerst mit VE-Wasser und dann mit unterschiedlichen Pufferlösungen befeuchtet. Die pH-Werte der Pufferlösungen entsprechen dem pH-Wert von menschlichem Urin (ca. pH 4,5-7,5).

Das Resultat ist in der folgenden Tabelle 2 dargestellt:

**Tabelle 2: Farbentwicklung des pH-Indikatorpapiers (Eintrag 4 aus Tabelle 1) nach Befeuchten mit vollentsalztem Wasser (VE-Wasser) und verschiedenen Pufferlösungen**

| **Eintrag** | **pH-Wert Puffer-Isg.** | **VE-Wasser** | **1 mM** | **5 mM** | **10 mM** | **20 mM** | **40 mM** | **80 mM** | **160 mM** |
|---|---|---|---|---|---|---|---|---|---|
| **1 ^{a)}** | 4,0 | Blau | Blau | Blau - sehr leicht Violett | Blau - sehr leicht Violett | leicht Violett | Violett | Violett - Magenta | Magenta |
| **2** ^{a)} | 5,0 | Blau | Blau | Blau - sehr leicht Violett | Blau - leicht Violett | Violett | Violett - Magenta | Magenta | Magenta |
| **3** ^{a)} | 6,0 | Blau | Blau | Blau - sehr leicht Violett | Blau - Violett | Violett | Magenta | Magenta | Magenta |
| **4 ^{b)}** | 7,0 | Blau | Blau | Blau - leicht Violett | Blau - Violett | Violett | Magenta | Magenta | Magenta |
| **5** ^{b)} | 8,0 | Blau | Blau | Blau - Violett | Violett | Magenta | Magenta | Magenta | Magenta |

| | | | | | | | | | |
|---|---|---|---|---|---|---|---|---|---|
| a) Zugabe von Pufferlösung auf Basis von Zitronensäure b) Zugabe von Pufferlösung auf Basis von Phosphatsalzen | | | | | | | | | |

Wie die Ergebnisse in der Tabelle 2 zeigen, ist eine Mindestkonzentration der Pufferlösung von 20 mM bei pH 4,0 nötig, um eine klare Farbreaktion zu erkennen (Eintrag 1). Der Farbumschlag ist bei höheren pH-Werten (Einträge 2-5) erwartungsgemäß früher zu erkennen (d.h. bei niedrigeren Pufferkonzentrationen von etwa 5-10 mM). Die Farbumschläge finden sofort statt und sind über mindestens 24 Stunden stabil.

Die Erfindung ist zudem in der folgenden Zeichnung dargestellt und nachfolgend beschrieben.

Es zeigen:

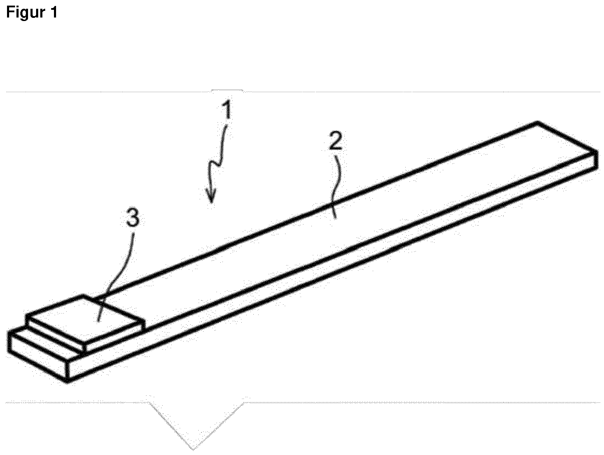

- Fig. 1: zeigt eine als Teststreifen 1 ausgebildete Testvorrichtung mit einem Kunststoffstreifen 2 als Träger, auf das eine Trägermatrix 3 befestigt ist. Die Trägermatrix enthält die für die Bestimmung notwendigen Nachweisreagenzien, die in bevorzugter Weise eine organische Säure und ein pH-Indikator sind.

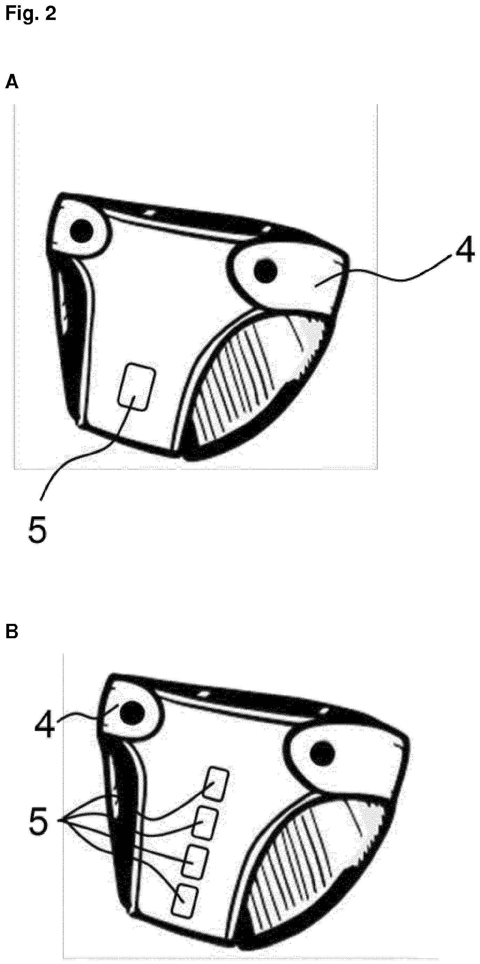

- Fig. 2: zeigt eine Einwegwindel 4, in die gemäß Fig. 2A eine Testvorrichtung 5 oder gemäß Fig. 2B mehrere Testvorrichtungen 5 eingearbeitet sind. Diese sind so angebracht, dass sie gewissermaßen als Testfenster von außen sichtbar sind und den Urin-Nachweis auch bei angezogener Windel erlauben. Das Vorsehen mehrerer Testvorrichtungen erlaubt über den qualitativen Urin-Nachweis hinaus einen quantitativen Urin-Nachweis, insofern die Anzahl der Urin-anzeigenden Testfenster eine Aussage über den Füllstatus der Windel erlaubt.

### Bezugszeichenliste

- 1: Teststreifen
- 2: Kunststoffstäbchen
- 3: Cellulosehaltige Trägermatrix mit immobilisiertem und Säure/Base-behandeltem pH-Indikatorfarbstoff
- 4: Einwegwindel
- 5: Testvorrichtung zum pH-induzierten Urin-Nachweis.

Weitere Varianten der Erfindung und ihre Ausführung ergeben sich für den Fachmann aus der vorangegangenen Offenbarung, den Figuren und den Patentansprüchen.

In den Patentansprüchen verwendete Begriffe wie "umfassen", "aufweisen", "beinhalten", "enthalten" und dergleichen schließen weitere Elemente oder Schritte nicht aus. Die Verwendung des unbestimmten Artikels schließt eine Mehrzahl nicht aus. Eine einzelne Einrichtung kann die Funktionen mehrerer in den Patentansprüchen genannten Einheiten bzw. Einrichtungen ausführen. In den Patentansprüchen angegebene Bezugszeichen sind nicht als Beschränkungen der eingesetzten Mittel und Schritte anzusehen.

## Patentansprüche

1. Testvorrichtung zum pH-abhängigen Nachweis von Urin enthaltend eine cellulosehaltige Trägermatrix bestehend aus:
a) einen pH-Indikatorfarbstoff mit einem Umschlagspunkt zwischen pH=0 und pH=4,5, der mittels einer chemischen Bindung an die Cellulose immobilisiert ist und mit einer Menge einer feststofflichen Säure so vorbehandelt ist, dass der pH-Indikatorfarbstoff im trockenen Zustand die Farbe des sauren pH-Bereiches anzeigt; oder
b) einen pH-Indikatorfarbstoff mit einem Umschlagspunkt zwischen pH=7,5 und pH=14, der mittels einer chemischen Bindung an die Cellulose immobilisiert ist und mit einer Menge einer feststofflichen Base so vorbehandelt ist, dass der pH-Indikatorfarbstoff im trockenen Zustand die Farbe des basischen pH-Bereiches anzeigt.
wobei der pH-Indikatorfarbstoff ein Reaktivfarbstoff ist mit mindestens einer reaktiven Gruppe zur kovalenten Anbindung an die Cellulose und zusätzlich zu der mindestens einen reaktiven Gruppe mindestens eine Sulfonsäure- und/oder Carbonsäuregruppe aufweist.

2. Testvorrichtung gemäß Anspruch 1, **dadurch gekennzeichnet, dass** die cellulosehaltige Trägermatrix ausgewählt ist aus der Gruppe bestehend aus Papier, bevorzugt Filterpapier; Karton; textiles Gewebe; textiles Vlies; und textiles Gewirke, wobei die cellulosehaltige Trägermatrix bevorzugt Filterpapier ist.

3. Testvorrichtung gemäß einem der vorangehenden Ansprüche, **dadurch gekennzeichnet, dass** der Reaktivfarbstoff ausgewählt ist aus der Gruppe bestehend aus Azofarbstoff, Anthrachinon-Farbstoff, Triphenylmethan-Farbstoff und Anthocyan-Farbstoff.

4. Testvorrichtung gemäß einem der vorangehenden Ansprüche, **dadurch gekennzeichnet, dass** die mindestens eine reaktive Gruppe ausgewählt ist aus der Gruppe bestehend aus 2-(Hydroxysulfonyloxy)-ethylsulfonyl, *N*-Methyl-*N*-[2-(hydroxysulfonyloxy)-ethyl]sulfonamido, Monochlortriazinyl, Dichlortriazinyl, Monochlorpyrimidyl, Dichlorpyrimidyl, Trichlorpyrimidyl, Tetrachlorpyrimidyl, Dichlorpyridazinyl, Trichlorpyridazinyl, Tetrachlorpyridazinyl, Dichlorchinoxalinyl, Dichlorphthalazinyl, 2-(Hydroxysulfonyloxy)ethylaminosulfonyl, 2,3-Dichlorchinoxalin-6-carbonsäure, 4-Chlorbenzolsulfonsäure [2-methoxyethylamid], 2-Chlorchinoxalin-6-carbonsäure, 3-Chlorchinoxalin-6-carbonsäure, 2,3-Dichlorchinoxalin-6-sulfonsäure, 2,3-Dichlorphthalazin-6-carbonsäure, 2,4-Dichlor-1,3,5-triazinyl, 2,4,6-Trichlor-1,3,5-triazinyl, 2,4-Dichlor-6-benzo-1,3,5-triazinyl, 2,4-Dichlor-6-amino-1,3,5-triazinyl und 3,5,6-Trichlor-1,2,4-triazinyl.

5. Testvorrichtung gemäß einem der vorangehenden Ansprüche, **dadurch gekennzeichnet, dass** die feststoffliche Säure ausgewählt ist aus der Gruppe bestehend aus Ascorbinsäure, Adipinsäure, Äpfelsäure, Agaricinsäure, Amidoschwefelsäure, 4-Aminosalicylsäure, Asparaginsäure, Bernsteinsäure, Benzolsulfonsäure, Benzoesäure, Borsäure, Caprinsäure, Cyclaminsäure, 2,2-Dichloressigsäure, Embonsäure, Ethansulfonsäure, Ethandisulfonsäure, Fumarsäure, Gentisinsäure, Gluconsäure, Glucuronsäure, Glutaminsäure, Glutarsäure, Glycerinsäure, Glycolsäure, Hippursäure, 2-Hydroxyethansulfonsäure, α-Ketoglutarsäure, Lactobionsäure, Laurinsäure, Maleinsäure, Malonsäure, Mandelsäure, Milchsäure, Mucinsäure, Natriumhydrogensulfat, Oxalessigsäure, Oxalsäure, Phthalsäure, 2-Phosphogylcerinsäure, 3-Phosphogylcerinsäure, Propionsäure, Polystyrolsulfonsäure, Pyroglutaminsäure, Pyrrolidin-2-carbonsäure, Salicylsäure, Sebacinsäure, Sorbinsäure, Sulfamsäure, *p-*Toluolsulfonsäure, Weinsäure, Zimtsäure und Zitronensäure, wobei Zitronensäure bevorzugt ist.

6. Testvorrichtung gemäß einem der Ansprüche 1 bis 4, **dadurch gekennzeichnet, dass** die feststoffliche Base ausgewählt ist aus der Gruppe bestehend aus Natriumhydroxid, Kaliumhydroxid, Lithiumhydroxid, Magnesiumhydroxid, Calciumhydroxid, Natriumphosphat, Natriumcarbonat und Natriumsulfid, wobei Natriumhydroxid bevorzugt ist.

7. Testvorrichtung gemäß einem der vorangehenden Ansprüche, **dadurch gekennzeichnet, dass** sie als Teststreifen, rechteckiges Testfeld oder Testband ausgebildet ist oder in ein integriertes Testsystem aufnehmbar ist.

8. Hygieneartikel umfassend eine oder mehrere Testvorrichtungen gemäß einem der Ansprüche 1 bis 7.

9. Hygieneartikel gemäß Anspruch 8, **dadurch gekennzeichnet, dass** der Hygieneartikel ausgewählt ist aus der Gruppe bestehend aus Windel, Windeleinlage, Inkontinenzartikel wie Inkontinenzeinlage, Inkontinenzauflage oder Inkontinenzhose und Bettauflage.

10. Verfahren zur Herstellung einer Testvorrichtung gemäß einem der Ansprüche 1 bis 7, umfassend die folgenden Schritte:
a) Bereitstellen einer cellulosehaltigen Trägermatrix mit einem durch chemische Bindung an die Cellulose immobilisierten pH-Indikatorfarbstoff, wobei der pH-Indikatorfarbstoff ein Reaktivfarbstoff ist mit mindestens einer reaktiven Gruppe zur kovalenten Anbindung an die Cellulose und zusätzlich zu der mindestens einen reaktiven Gruppe mindestens eine Sulfonsäure- und/oder Carbonsäuregruppe aufweist.
b) Imprägnieren der farbstofftragenden Cellulose-Trägermatrix aus Schritt a) durch Durchtränken mit einer Imprägnierlösung, die Wasser, Alkohol und eine feststoffliche Base oder eine feststoffliche Säure enthält,
c) Trocknen der farbstofftragenden Cellulose-Trägermatrix aus Schritt b),
d) optionales Zurechtschneiden der getrockneten Trägermatrix aus Schritt c) auf die gewünschte Größe.

11. Verfahren gemäß Anspruch 10, **dadurch gekennzeichnet, dass** die Imprägnierlösung die Säure oder Base in einer Konzentration zwischen 2 mM und 75 mM, bevorzugt zwischen 5 und 60 mM und besonders bevorzugt zwischen 10 und 30 mM enthält.

12. Verfahren gemäß Anspruch 10, **dadurch gekennzeichnet, dass** das Trocknen im Schritt c) bei einer Temperatur von mehr als 25 °C und bevorzugt bei einer Temperatur zwischen 250 und 350 °C erfolgt.

## Claims

1. A test device for the pH-dependent detection of urine, containing a cellulose-containing support matrix consisting of:
a) a pH indicator dye with a transfer point of between pH = 0 and pH = 4.5, immobilized to the cellulose by means of a chemical bond, and pretreated with a quantity of a solid acid so that the pH indicator dye indicates the color of the acidic pH range when in a dry state; or
b) a pH indicator dye with a transfer point of between pH = 7.5 and pH = 14, immobilized to the cellulose by means of a chemical bond, and pretreated with a quantity of a solid base so that the pH indicator dye indicates the color of the basic pH range when in a dry state,
wherein said pH indicator dye is a reactive dye having at least one reactive group for covalent bonding to the cellulose, and has at least one sulfonic acid and/or carboxylic acid group in addition to said at least one reactive group.

2. The test device according to claim 1, **characterized in that** said cellulose-containing support matrix is selected from the group consisting of paper, preferably filter paper; cardboard; textile fabric; textile non-woven; and textile knitting, wherein said cellulose-containing support matrix is preferably made of filter paper.

3. The test device according to any of the preceding claims, **characterized in that** said reactive dye is selected from the group consisting of azo dye, anthraquinone dye, triphenylmethane dye, and anthocyan dye.

4. The test device according to any of the preceding claims, **characterized in that** said at least one reactive dye is selected from the group consisting of 2-(hydroxysulfonyloxy)-ethylsulfonyl, *N*-methyl-*N*-[2-(hydroxysulfonyloxy)-ethyl]sulfonamido, monochlorotriazinyl, dichlorotriazinyl, monochloropyrimidyl, dichloropyrimidyl, trichloropyrimidyl, tetrachloropyrimidyl, dichloropyridazinyl, trichloropyridazinyl, tetrachloropyridazinyl, dichloroquinoxalinyl, dichlorophthalazinyl, 2-(hydroxysulfonyloxy)ethylaminosulfonyl, 2,3-dichloroquinoxaline-6-carboxylic acid, 4-chlorobenzenesulfonic acid [2-methoxyethyl amide], 2-chloroquinoxaline-6-carboxylic acid, 3-chloroquinoxaline-6-carboxylic acid, 2,3-dichloroquinoxaline-6-sulfonic acid, 2,3-dichlorophthalazine-6-carboxylic acid, 2,4-dichloro-1,3,5-triazinyl, 2,4,6-trichloro-1,3,5-triazinyl, 2,4-dichloro-6-benzo-1,3,5-triazinyl, 2,4-dichloro-6-amino-1,3,5-triazinyl, and 3,5,6-trichloro-1,2,4-triazinyl.

5. The test device according to any of the preceding claims, **characterized in that** said solid acid is selected from the group consisting of ascorbic acid, adipic acid, malic acid, agaric acid, amidosulfuric acid, 4-aminosalicylic acid, aspartic acid, succinic acid, benzenesulfonic acid, benzoic acid, boric acid, capric acid, cyclamic acid, 2,2-dichloroacetic acid, embonic acid, ethanesulfonic acid, ethanedisulfonic acid, fumaric acid, gentisic acid, gluconic acid, glucuronic acid, glutamic acid, glutaric acid, glyceric acid, glycolic acid, hippuric acid, 2-hydroxyethanesulfonic acid, α-ketoglutaric acid, lactobionic acid, lauric acid, maleic acid, malonic acid, mandelic acid, lactic acid, mucic acid, sodium hydrogensulfate, oxaloacetic acid, oxalic acid, phthalic acid, 2-phosphoglyceric acid, 3-phosphoglyceric acid, propionic acid, polystyrenesulfonic acid, pyroglutamic acid, pyrrolidine-2-carboxylic acid, salicylic acid, sebacic acid, sorbic acid, sulfamic acid, p-toluenesulfonic acid, tartaric acid, cinnamic acid, and citric acid, wherein citric acid is preferred.

6. The test device according to any of claims 1 to 4, **characterized in that** said solid base is selected from the group consisting of sodium hydroxide, potassium hydroxide, lithium hydroxide, magnesium hydroxide, calcium hydroxide, sodium phosphate, sodium carbonate, and sodium sulfide, wherein sodium hydroxide is preferred.

7. The test device according to any of the preceding claims, **characterized by** being formed as a test strip, rectangular test pad, or test tape, or includable in an integrated test system.

8. A sanitary product, comprising one or more test devices according to any of claims 1 to 7.

9. The sanitary product according to claim 8, **characterized in that** said sanitary product is selected from the group consisting of diaper, napkin liner, incontinence articles, such as an incontinence pad, incontinence topper or incontinence pants, and mattress pad.

10. A process for preparing a test device according to any of claims 1 to 7, comprising the following steps:
a) providing a cellulose-containing support matrix with a pH indicator dye immobilized to the cellulose by means of a chemical bond, wherein said pH indicator dye is a reactive dye having at least one reactive group for covalent bonding to the cellulose, and has at least one sulfonic acid and/or carboxylic acid group in addition to said at least one reactive group,
b) impregnating said dye-containing cellulose support matrix from step a) by soaking with an impregnating solution containing water, alcohol, and a solid base or a solid acid,
c) drying the dye-containing cellulose support matrix from step b),
d) optionally trimming the dried support matrix from step c) to the desired size.

11. The process according to claim 10, **characterized in that** said impregnating solution contains said acid or base in a concentration of between 2 mM and 75 mM, preferably between 5 and 60 mM, and more preferably between 10 and 30 mM.

12. The process according to claim 10, **characterized in that** said drying in step c) is effected at a temperature of more than 25 °C, preferably at a temperature of between 250 and 350 °C.

## Revendications

1. Dispositif de test pour une détection dépendante du pH d'urine, contenant une matrice de support contenant de la cellulose et consistant en :
a) un colorant indicateur de pH présentant un point de transfert compris entre pH=0 et pH=4,5, immobilisé par l'intermédiaire d'une liaison chimique à la cellulose et prétraité avec une quantité d'un acide solide de telle manière que le colorant indicateur de pH, à l'état sec, indique la couleur de la plage de pH acide ; ou
b) un colorant indicateur de pH présentant un point de transfert compris entre pH=7,5 et pH=14, immobilisé par l'intermédiaire d'une liaison chimique à la cellulose et prétraité avec une quantité d'une base solide de telle manière que le colorant indicateur de pH, à l'état sec, indique la couleur de la plage de pH basique ;
le colorant indicateur de pH étant un colorant réactif avec au moins un groupe réactif pour une liaison covalente à la cellulose et, en plus dudit au moins un groupe réactif, comprend au moins un groupe acide sulfonique et/ou acide carboxylique.

2. Dispositif de test selon la revendication 1, **caractérisé en ce que** la matrice de support cellulosique est choisie dans le groupe constitué par du papier, de préférence du papier filtre ; du carton ; du tissu textile ; du tissu textile non-tissé ; et du tissu textile tricoté, la matrice de support cellulosique étant de préférence du papier filtre.

3. Dispositif de test selon l'une quelconque des revendications précédentes, **caractérisé en ce que** le colorant réactif est choisi dans le groupe constitué par un colorant azoïque, un colorant anthraquinone, un colorant triphénylméthane et un colorant anthocyane.

4. Dispositif de test selon l'une quelconque des revendications précédentes, **caractérisé en ce que** le au moins un groupe réactif est choisi dans le groupe comprenant 2-(hydroxysulfonyloxy)-éthylsulfonyle, *N*-méthyl-*N*-[2-(hydroxysulfonyloxy)-éthyl]sulfonamido, monochlorotriazinyle, dichlorotriazinyle, monochloropyrimidyle, dichloropyrimidyle, trichloropyrimidyle, tétrachloropyrimidyle, dichloropyridazinyle, trichloropyridazinyle, tétrachloropyridazinyle, dichloroquinoxalinyle, dichlorophtalazinyle, 2-(hydroxysulfonyloxy)éthylaminosulfonyle, acide 2,3-dichloroquinoxaline-6-carboxylique, acide 4-chlorobenzènesulfonique de [2-méthoxyéthylamide], acide 2-chloroquinoxaline-6-carboxylique, acide 3-chloroquinoxaline-6-carboxylique, acide 2,3-dichloroquinoxaline-6-sulfonique, acide 2,3-dichlorophtalazine-6-carboxylique, 2,4-dichloro-1,3,5-triazinyle, 2,4,6-trichloro-1,3,5-triazinyle, 2,4-dichloro-6-benzo-1,3,5-triazinyle, 2,4-dichloro-6-amino-1,3,5-triazinyle et 3,5,6-trichloro-1,2,4-triazinyle.

5. Dispositif de test selon l'une quelconque des revendications précédentes, **caractérisé en ce que** l'acide solide est choisi dans le groupe comprenant acide ascorbique, acide adipique, acide malique, acide agarique, acide sulfamique, acide 4-aminosalicylique, acide aspartique, acide succinique, acide benzènesulfonique, acide benzoïque, acide borique, acide caprique, acide cyclamique, acide 2,2-dichloroacétique, acide embonique, acide éthanesulfonique, acide éthanedisulfonique, acide fumarique, acide gentisique, acide gluconique, acide glucuronique, acide glutamique, acide glutarique, acide glycérique, acide glycolique, acide hippurique, acide 2-hydroxyéthanesulfonique, acide α-cétoglutarique, acide lactobionique, acide laurique, acide maléique, acide malonique, acide mandélique, acide lactique, acide mucinique, hydrogénosulfate de sodium, acide oxaloacétique, acide oxalique, acide phtalique, acide 2-phosphoglycérique, acide 3-phosphoglycérique, acide propionique, acide polystyrène sulfonique, acide pyroglutamique, acide pyrrolidine-2-carboxylique, acide salicylique, acide sébacique, acide sorbique, acide sulfamique, acide *p*-toluènesulfonique, acide tartrique, acide cinnamique et l'acide citrique, l'acide citrique étant préféré.

6. Dispositif de test selon l'une quelconque des revendications 1 à 4, **caractérisé en ce que** la base solide est choisie dans le groupe comprenant l'hydroxyde de sodium, l'hydroxyde de potassium, l'hydroxyde de lithium, l'hydroxyde de magnésium, l'hydroxyde de calcium, le phosphate de sodium, le carbonate de sodium et le sulfure de sodium, l'hydroxyde de sodium étant préféré.

7. Dispositif de test selon l'une quelconque des revendications précédentes, **caractérisé en ce qu'**il se présente sous la forme de bandelette de test, de champ de test rectangulaire ou de bande de test ou peut être logé dans un système de test intégré.

8. Article d'hygiène comprenant un ou plusieurs dispositifs de test selon l'une quelconque des revendications 1 à 7.

9. Article d'hygiène selon la revendication 8, **caractérisé en ce que** l'article d'hygiène est choisi parmi le groupe comprenant couche, doublure de couche, articles pour incontinence comme doublure pour incontinence, coussinet pour incontinence ou pantalon pour incontinence et alèse.

10. Procédé de fabrication d'un dispositif de test selon l'une quelconque des revendications 1 à 7, comprenant les étapes suivantes consistant à :
a) préparer une matrice de support contenant de la cellulose avec un colorant indicateur de pH immobilisé par liaison chimique à la cellulose, le colorant indicateur de pH étant un colorant réactif comprenant au moins un groupe réactif pour une liaison covalente à la cellulose et comprenant en plus dudit au moins un groupe réactif au moins un groupe acide sulfonique et/ou acide carboxylique.
b) imprégner la matrice de support cellulosique porteuse de colorant de l'étape a) en l'imprégnant d'une solution d'imprégnation contenant de l'eau, de l'alcool et une base solide ou un acide solide ;
c) sécher la matrice de support cellulosique porteuse de colorant de l'étape b),
d) découper facultativement la matrice de support séchée de l'étape c) à la taille souhaitée.

11. Procédé selon la revendication 10, **caractérisé en ce que** la solution d'imprégnation contient l'acide ou la base à une concentration comprise entre 2 mM et 75 mM, de préférence comprise entre 5 et 60 mM et de manière particulièrement préférée comprise entre 10 et 30 mM.

12. Procédé selon la revendication 10, **caractérisé en ce que** le séchage à l'étape c) a lieu à une température supérieure à 25 °C et de préférence à une température comprise entre 250 et 350 °C.
